# EUROPEAN PATENT SPECIFICATION

(11) **EP 3 485 797 B1**
(45) Date of publication and mention of the grant of the patent: **03.06.2020**
(21) Application number: 17201857.4
(22) Date of filing: 15.11.2017
(51) Int. Cl.: A61B 1/06, A61B 1/31, A61B 1/00

(54) **SIGMOIDOSCOPE WITH INTEGRAL ILLUMINATING ASSEMBLY**
SIGMOIDOSKOP MIT INTEGRALER BELEUCHTUNGSANORDNUNG
SIGMOÏDOSCOPE AVEC ENSEMBLE D'ÉCLAIRAGE INTÉGRAL

(43) Date of publication of application: 22.05.2019
(73) Proprietor: Tsai, Yih-Chiou, Taichung (TW)
(72) Inventor: Tsai, Yih-Chiou, Taichung (TW)
(74) Representative: Cabinet Chaillot

(56) References cited:
- AU-B1- 2017 261 460
- CN-Y- 2 166 771
- GB-A- 1 415 024
- GB-A- 2 493 165
- US-A1- 2015 238 070

## Description

The present invention relates to a medical instrument, and more particularly to a sigmoidoscope with an integral illuminating assembly.

### 2. Description of Related Art

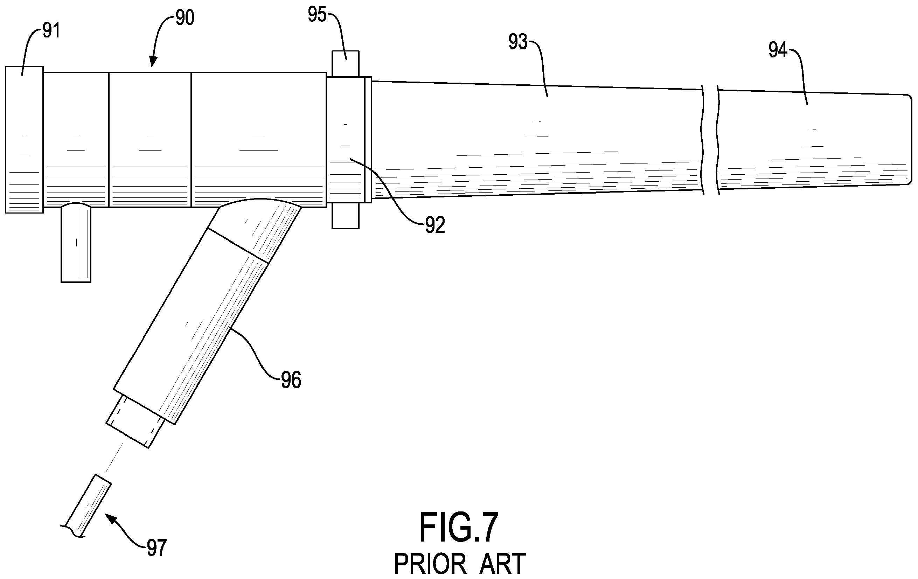

With reference to Fig. 7, a conventional sigmoidoscope has an optic head 90 with a front end and a rear end, an eyepiece 91 disposed at the rear end of the optic head 90, and a coupling mount 92 disposed at the front end of the optic head 90. The coupling mount 92 is designed for assembling a disposable speculum 93. The speculum 93 is light-permeable and has a guiding tube 94. A spigot 95 is disposed on the coupling mount 92 for introducing fluid to clean inside of an instrument. A handle 96 is connected to the optic head 90 for assembling an illuminating assembly. The illuminating assembly may be an optic fiber 97 and is inserted in the handle 96 for illumination.

Light emitted by the illuminating assembly assembled in the handle 96 is refracted by a prism and then projected to the guiding tube 94 of the speculum 93 for illumination when the conventional sigmoidoscope is being inserted into a human body.

The conventional sigmoidoscope has drawbacks as follows:
1. The illuminating assembly assembled in the handle 96 has to be manually turned on or off by the medical personnel and is inconvenient for operating as the medical personnel is simultaneously engaged in administration of medication or other clinical activities.
2. Light emitted by the illuminating assembly assembled in the handle 96 projected to the guiding tube 94 of the speculum 93 is refracted by the prism at first. Therefore, the conventional sigmoidoscope has a problem of uneven illumination and is defective in inspection.

An example of such sigmoidoscope can be found in the patent document GB2493165A.

To overcome the shortcomings of the conventional sigmoidoscope, the present invention provides a sigmoidoscope with an integral illuminating assembly to mitigate or obviate the aforementioned problems.

The main objective of the present invention is to provide a sigmoidoscope with an integral illuminating assembly that is able to simultaneously emit light when the speculum assembly is being assembled.

The sigmoidoscope comprises a main body and an illuminating assembly. The main body has an optic head. The optic head has a channel extending through the optic head and a positioning recess disposed at a middle portion of the optic head. The illuminating assembly has a circuit board, multiple light emitting elements, and a tact switch. The circuit board is disposed in the positioning recess and has a central hole aligned with the channel. The multiple light emitting elements are annularly mounted to the circuit board at intervals and are electrically connected to the circuit board. The tact switch is mounted to the circuit board, is electrically connected to the circuit board, and has a pushing button extending over a rim of the central hole. The pushing button can be pressed by an inserting tube of a speculum assembly that is inserted into the central hole.

Other objects, advantages, and novel features of the invention will become more apparent from the following detailed description when taken in conjunction with the accompanying drawings.

### In the drawings

Fig. 1 is a perspective view of a sigmoidoscope with an integral illuminating assembly in accordance with the present invention;
Fig. 2 is an exploded perspective view of the sigmoidoscope in Fig. 1;
Fig. 3 is a side view in partial section of an optic head of the sigmoidoscope in Fig. 1;
Fig. 4 is an enlarged side view in partial section of the sigmoidoscope in Fig. 1, showing an inserting tube being inserted through a circuit board;
Fig. 5 is a side view in partial section of the sigmoidoscope in Fig. 1;
Fig. 6 is a partial perspective view of an illuminating assembly in Fig. 2, showing the circuit board having multiple surface mount device light emitting diodes mounted thereon; and
Fig. 7 is a perspective view of a sigmoidoscope in accordance with the prior art.

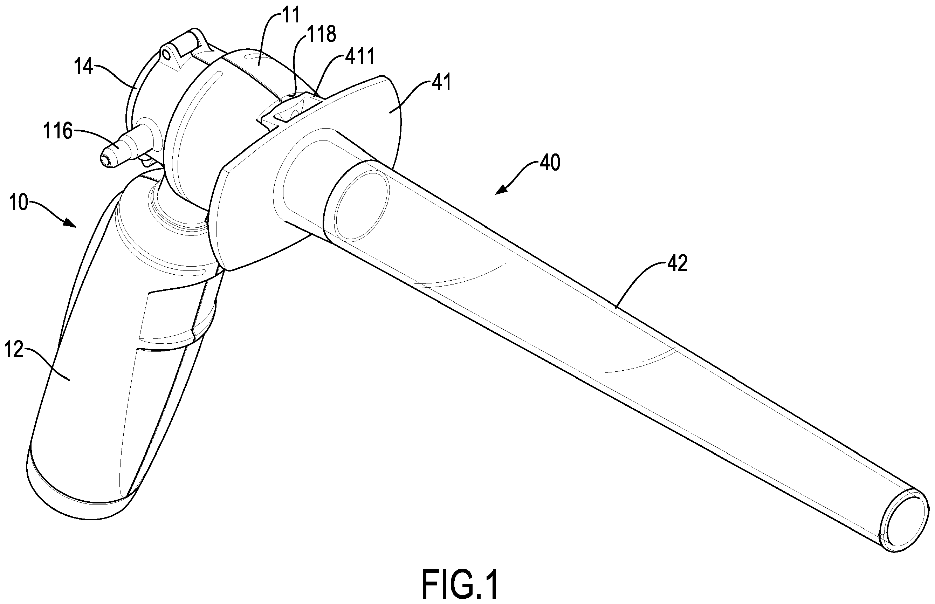

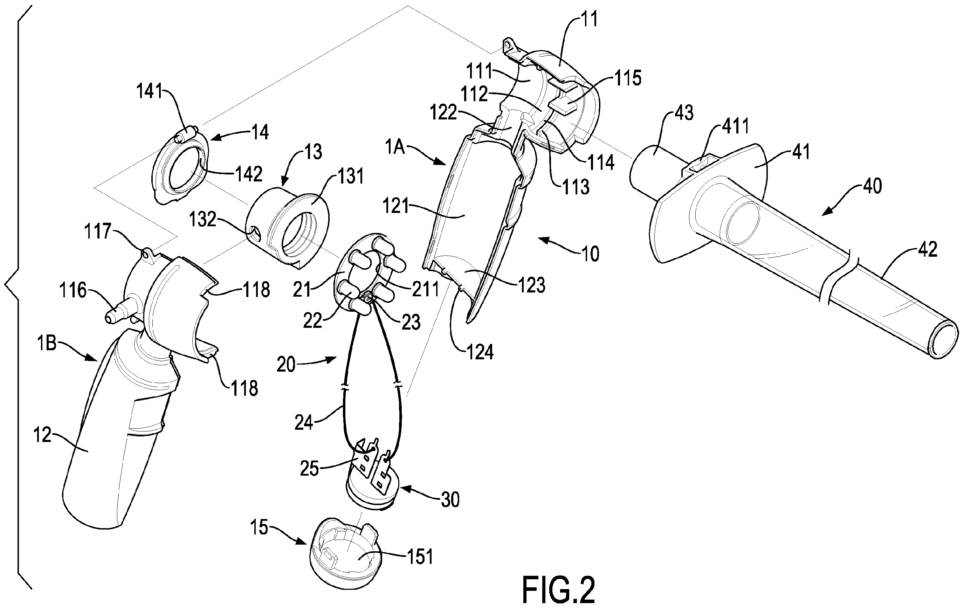

With reference to Figs. 1 and 2, a sigmoidoscope with an integral illuminating assembly in accordance with the present invention has a main body 10, an illuminating assembly 20 mounted in the main body 10, and a speculum assembly 40 mounted to the main body 10. The sigmoidoscope further has a coupling sleeve 13, an eyepiece 14, and a cap 15.

The main body 10 may be integrally formed as a single piece or be symmetrically composed by two shells 1A, 1B. In the embodiment of the present invention, the main body 10 is symmetrically assembled by the two shells 1A, 1B. The main body 10 has an optic head 11 and a handle 12 connected to the optic head 11. The coupling sleeve 13 is embedded in the optic head 11. The eyepiece 14 is connected to a rear end of the optic head 11. The cap 15 is connected to a lower end of the handle 12.

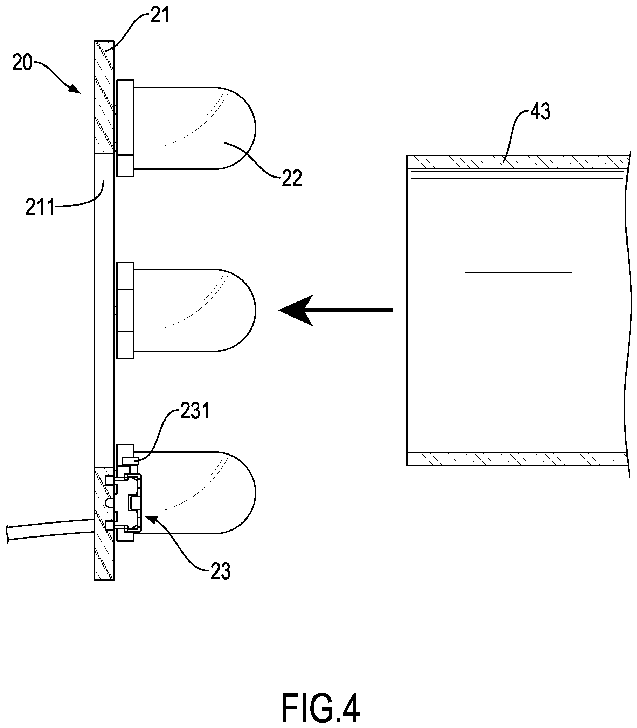

With reference to Figs. 2, 3, and 4, the optic head 11 is tubular and has an axis, a front end, the rear end, a channel 111, a first section, a second section, a connecting section 112, an annular rib 113, a positioning recess 114, multiple positioning plates 115, a spigot 116, a pivoting mount 117, and two engaging indentions 118. The front end and the rear end of the optic head 11 are opposite along the axis of the optic head 11. The channel 111 extends through the optic head 11 along the axis of the optic head 11. The first section, the second section, and the connecting section 112 of the optic head 11 are aligned along the axis of the optic head 11. The second section of the optic head 11 has an internal diameter larger than an internal diameter of the first section of the optic head 11. The connecting section 112 is disposed between the first and the second sections of the optic head 11. The connecting section 112 is connected to both the first section and the second section of the optic head 11.

The annular rib 113 is formed on an internal surface of the first section of the optic head 11. The positioning recess 114 is collectively formed by the first section, the connecting section 112, and the annular rib 113 of the optic head 11. The positioning recess 114 is disposed between the connecting section 112 and the annular rib 113. The multiple positioning plates 115 are formed on the internal surface of the first section of the optic head 11. The multiple positioning plates 115 are disposed along the annular rib 113 at intervals. Each one the multiple positioning plates 115 has a rear side facing to the second section of the optic head 11. The spigot 116 is formed on the second section of the optic head 11. The pivoting mount 117 is formed on the second section of the optic head 11 and disposed at the rear end of the optic head 11. The two engaging indentions 118 are formed in the first section of the optic head 11 and disposed at the front end of the optic head 11.

The handle 12 is hollow, is connected to the optic head 11, and has a chamber 121, a tunnel 122, and an assembling mount 123. The chamber 121 is formed in the handle 12 and communicates with the channel 111 via the tunnel 122. The assembling mount 123 is adjacent to the lower end of the handle 12 and has two engaging protrusions 124 spaced from each other.

The coupling sleeve 13 with flexibility is inserted in the channel 111 of the optic head 11, is aligned with the channel 111, and has an annular abutting portion 131 and a through hole 132. The annular abutting portion 131 is fitted in the positioning recess 114 to secure the coupling sleeve 13 embedded in the optic head 11. The through hole 132 is formed through the coupling sleeve 13. The spigot 116 communicates with the channel 111 via the through hole 132.

The eyepiece 14 is made of transparent materials and is pivotally connected to the rear end of the optic head 11. The eyepiece 14 is able to cover the rear end of the optic head 11. The eyepiece 14 has a pivoting shaft 141 and a lens portion 142. The pivoting shaft 141 is designed for connecting the pivoting mount 117. The lens portion 142 with magnification function has a diameter determined in accordance with a cross-section of the channel 111 of the optic head 11.

The cap 15 engages with and is connected to the lower end of the handle 12. The cap 15 covers the lower end of the handle 12 and has a receiving recess 151 disposed within the cap 15.

With reference to Figs. 2, 3, and 4, the illuminating assembly 20 has a circuit board 21, multiple light emitting elements 22, a tact switch 23, two wires 24, two electrodes 25, and at least one battery 30. The circuit board 21 is disposed in the positioning recess 114 and has an annular outline and a central hole 211. The central hole 211 is aligned with the channel 111. The multiple light emitting elements 22 are annularly mounted to the circuit board 21 at intervals and face to the front end of the optic head 11. The multiple light emitting elements 22 are electrically connected to the circuit board 21. The multiple light emitting elements 22 are six in amount. The six light emitting elements 22 may be dual in-line package light emitting diodes or surface mount device light emitting diodes.

The tact switch 23 is mounted to the circuit board 21 for turning on or off the six light emitting elements 22. The tact switch 23 has a pushing button 231 extending over a rim of the central hole 211. The two wires 24 are electrically connected to the circuit board 21, pass through the tunnel 122, and extend into the chamber 121. The two electrodes 25 are respectively connected to the two wires 24, and are respectively engaged with the two engaging protrusions 124 to be fastened to the assembling mount 123. The at least one battery 30 is held in the receiving recess 151, contacts the two electrodes 25, and is electrically connected to the two electrodes 25. In the embodiment of the present invention, the at least one battery 30 is two in amount. The two batteries 30 are button cell batteries.

The speculum assembly 40 has a blocking unit 41, a speculum 42, an inserting tube 43 connected to the speculum 42, and an inspection channel disposed along an axis of the speculum assembly 40. The speculum 42 is light-permeable.

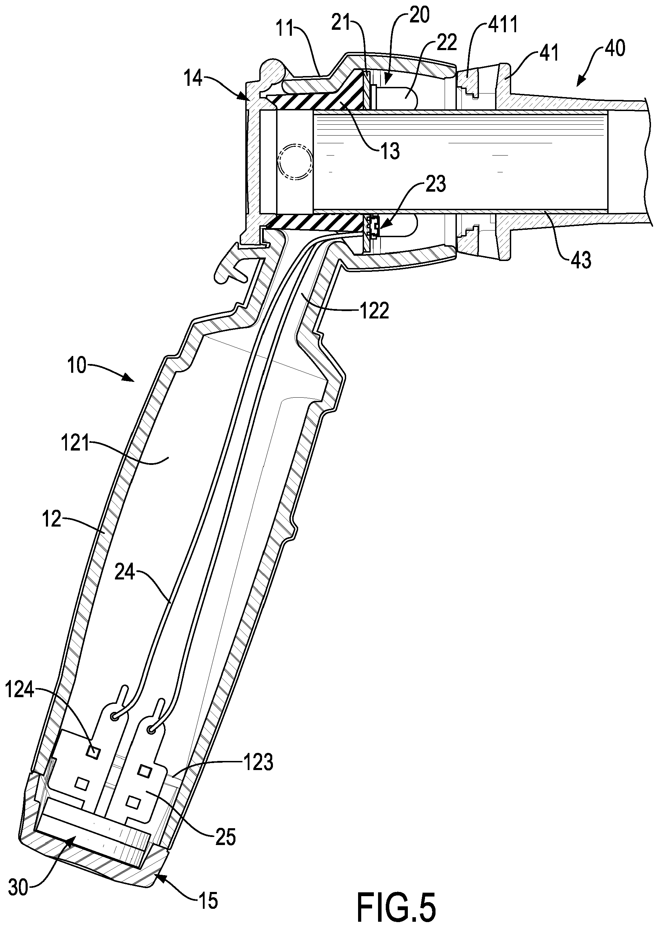

The blocking unit 41 of the speculum assembly 40 has two engaging portions 411. The speculum 42 is integrally formed on the blocking unit 41. The inserting tube 43 is opaque and is connected to the blocking unit 41. The inserting tube 43 is inserted into the speculum 42 through the blocking unit 41. The blocking unit 41 is disposed between the speculum 42 and the inserting tube 43. With reference to Figs. 1 and 5, the speculum assembly 40 is mounted to the main body 10. The two engaging portions 411 respectively engage with the two engaging indentions 118 to secure the speculum assembly 40 onto the main body 10. With reference to Figs. 1, 2, and 5, the inserting tube 43 passes through the circuit board 21 and then is inserted into the coupling sleeve 13. Since the coupling sleeve 13 is made of a flexible material, the inserting tube 43 may be securely mounted to the optic head 11 conveniently.

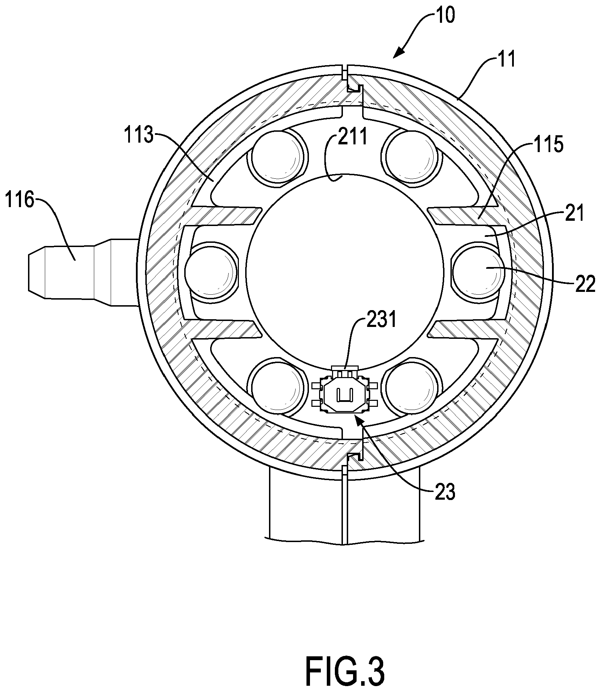

With reference to Figs. 3, 4, and 5, the pushing button 231 of the tact switch 23 of the illuminating assembly 20 extends into the central hole 211 of the circuit board 21. When the inserting tube 43 passes through the circuit board 21 and presses the pushing button 231, the tact switch 23 turns on the six light emitting elements 22 to emit light.

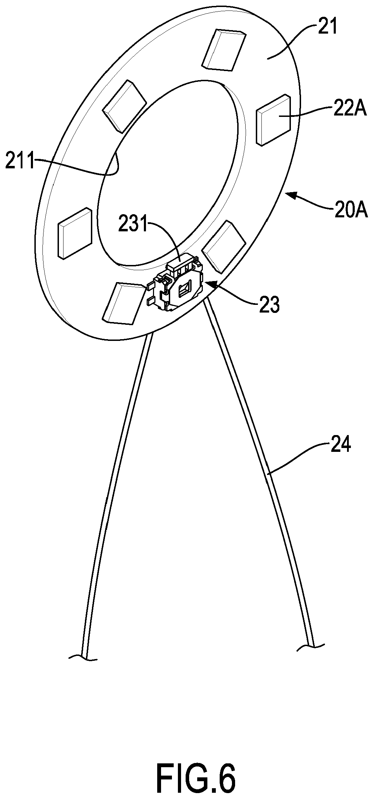

With reference to Fig. 6, the six light emitting elements 22 are surface mount device light emitting diodes.

With reference to Figs. 1 to 5, the central hole 211 of the circuit board 21 of the illuminating assembly 20 is aligned with the channel 111 of the optic head 11. The pushing button 231 extends over the rim of the central hole 211 of the circuit board 21 and extends into the central hole 211. When the inserting tube 43 of the speculum assembly 40 is inserted into the coupling sleeve 13 to mount to the main body 10, the six light emitting elements 22 are turned on simultaneously and manual switching is not needed. The six light emitting elements 22 annularly mounted to the circuit board 21 with the opaque inserting tube 43 allow light emitted by the six light emitting elements 22 to be evenly emitted to the speculum 42 of the speculum assembly 40. The sigmoidoscope in accordance with the present invention provides an even and bright illumination.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. A sigmoidoscope comprising:
a main body (10) having
an optic head (11) having
a channel (111) extending through the optic head (11); and
a positioning recess (114) disposed at a middle portion of the optic head (11);
a handle (12) connected to the optic head (11) and having a chamber (121) communicating with the channel (111); and
an illuminating assembly (20) having
a circuit board (21) disposed in the positioning recess (114) and having
an annular outline; and
a central hole (211) aligned with the channel (111);
multiple light emitting elements (22,22A) annularly mounted to the circuit board (21) at intervals and electrically connected to the circuit board (21);
two wires (24) electrically connected to the circuit board (21);
two electrodes (25) respectively and electrically connected to the two wires (24); and
at least one battery (30) electrically connecting the two electrodes (25), the sigmoidoscope being further **characterized by** having a tact switch (23) mounted to the circuit board (21), electrically connected to the circuit board (21) and by having a pushing button (231) extending over a rim of the central hole (211) and being able to be pressed by an inserting tube (43) of a speculum assembly (40) that is inserted into the central hole (211).

2. The sigmoidoscope as claimed in claim 1, wherein the sigmoidoscope has
a coupling sleeve (13) with flexibility, aligned with the channel (111) and having an annular abutting portion (131); and
the annular abutting portion (131) is embedded in the positioning recess (114) of the optic head (11) and is adjacent to the circuit board (21).

3. The sigmoidoscope as claimed in claim 2, wherein the sigmoidoscope has
an eyepiece (14) pivotally connected to the optic head (11) and having a lens portion (142) aligned with the channel (111);
the coupling sleeve (13) is disposed between the eyepiece (14) and the circuit board (21).

4. The sigmoidoscope as claimed in claim 3, wherein
the main body (10) is symmetrically composed by two shells (1A,1B).

5. The sigmoidoscope as claimed in claim 4, wherein the sigmoidoscope has
a cap (15) covering an end of the handle (12) distal from the optic head (11) and having a receiving recess (151) for holding the at least one battery (30); and
the handle (12) has an assembling mount (123) for locating the two electrodes (25), the assembling mount (123) disposed adjacent to the chamber (121) and being distal from the optic head (11).

6. The sigmoidoscope as claimed in claim 5, wherein the optic head (11) has at least one engaging indention (118) disposed distal from the eyepiece (14).

7. The sigmoidoscope as claimed in any one of claims 1 to 6, wherein
the optic head (11) has
a first section;
a second section having an internal diameter larger than an internal diameter of the first section;
a connecting section (112) disposed between the first section and the second section; and
an annular rib (113) disposed adjacent to the connecting section (112); and
the positioning recess (114) is disposed between the connecting section (112) and the annular rib (113).

8. The sigmoidoscope as claimed in claim 7, wherein the optic head (11) has
multiple positioning plates (115) disposed along the annular rib at intervals, and each one of the multiple positioning plates has a rear side facing to the eyepiece and abutted by the circuit board.

9. The sigmoidoscope as claimed in claim 8, wherein the multiple light emitting elements (22,22A) are dual in-line package light emitting diodes.

10. The sigmoidoscope as claimed in claim 8, wherein the multiple light emitting elements (22,22A) are surface mount device light emitting diodes.

## Patentansprüche

1. Sigmoidoskop, umfassend:
einen Hauptkörper (10), der aufweist
einen Optikkopf (11), der aufweist
einen Kanal (111), der sich durch den Optikkopf (11) erstreckt; und
eine Positionierungsaussparung (114), die in einem mittleren Abschnitt des Optikkopfes (11) angeordnet ist;
einen Griff (12), der mit dem Optikkopf (11) verbunden ist und eine Kammer (121) aufweist, die mit dem Kanal (111) in Verbindung steht; und
eine Beleuchtungsbaugruppe (20), die aufweist
eine Leiterplatte (21), die in der Positionierungsaussparung (114) angeordnet ist und aufweist
einen ringförmigen Umriss; und
ein Mittelloch (211), das auf den Kanal (111) ausgerichtet ist;
mehrere Leuchtelemente (22, 22A), die in Abständen ringförmig an der Leiterplatte (21) montiert und mit der Leiterplatte (21) elektrisch verbunden sind;
zwei Drähte (24), die mit der Leiterplatte (21) elektrisch verbunden sind;
zwei Elektroden (25), die jeweils mit den beiden Drähten (24) elektrisch verbunden sind; und
mindestens eine Batterie (30), welche die beiden Elektroden (25) elektrisch verbindet,
wobei das Sigmoidoskop ferner **dadurch gekennzeichnet ist, dass** es einen TACT-Schalter (23) aufweist, der an der Leiterplatte (21) montiert und mit der Leiterplatte (21) elektrisch verbunden ist, und dass es einen Druckknopf (231) aufweist, der sich über einen Rand des Mittellochs (211) erstreckt und durch eine Einsetzröhre (43) einer Spekulum-Baugruppe (40), die in das Mittelloch (211) eingesetzt wird, gedrückt werden kann.

2. Sigmoidoskop nach Anspruch 1, wobei das Sigmoidoskop aufweist
eine flexible Kupplungsmuffe (13), die auf den Kanal (111) ausgerichtet ist und einen ringförmigen Stoßabschnitt (131) aufweist; und
wobei der ringförmige Stoßabschnitt (131) in der Positionierungsaussparung (114) des Optikkopfes (11) eingebettet ist und an die Leiterplatte (21) angrenzt.

3. Sigmoidoskop nach Anspruch 2, wobei das Sigmoidoskop aufweist
ein Okular (14), das schwenkbar mit dem Optikkopf (11) verbunden ist und einen Linsenabschnitt (142) aufweist, der auf den Kanal (111) ausgerichtet ist;
wobei die Kupplungsmuffe (13) zwischen dem Okular (14) und der Leiterplatte (21) angeordnet ist.

4. Sigmoidoskop nach Anspruch 3, wobei der Hauptkörper (10) symmetrisch aus zwei Schalen (1A, 1B) besteht.

5. Sigmoidoskop nach Anspruch 4, wobei das Sigmoidoskop aufweist
eine Kappe (15), die ein zu dem Optikkopf (11) distales Ende des Griffs (12) abdeckt und eine Aufnahmeaussparung (151) zum Halten der mindestens einen Batterie (30) aufweist; und
der Griff (12) eine Montagehalterung (123) aufweist, um die beiden Elektroden (25) anzuordnen, wobei die Montagehalterung (123) neben der Kammer (121) angeordnet ist und zu dem Optikkopf (11) distal ist.

6. Sigmoidoskop nach Anspruch 5, wobei der Optikkopf (11) mindestens eine Eingriffsvertiefung (118) aufweist, die zu dem Okular (14) distal angeordnet ist.

7. Sigmoidoskop nach einem der Ansprüche 1 bis 6, wobei
der Optikkopf (11) aufweist
einen ersten Teilabschnitt;
einen zweiten Teilabschnitt, der einen Innendurchmesser aufweist, der größer als ein Innendurchmesser des ersten Teilabschnitts ist;
einen Verbindungsteilabschnitt (112), der zwischen dem ersten Teilabschnitt und dem zweiten Teilabschnitt angeordnet ist; und
eine ringförmige Rippe (113), die neben dem Verbindungsteilabschnitt (112) angeordnet ist; und
die Positionierungsaussparung (114) zwischen dem Verbindungsteilabschnitt (112) und der ringförmigen Rippe (113) angeordnet ist.

8. Sigmoidoskop nach Anspruch 7, wobei der Optikkopf (11) mehrere Positionierungsplatten (115) aufweist, die in Abständen entlang der ringförmigen Rippe angeordnet sind, und jede der mehreren Positionierungsplatten eine Rückseite aufweist, die dem Okular zugewandt ist und an die Leiterplatte anstößt.

9. Sigmoidoskop nach Anspruch 8, wobei die mehreren Leuchtelemente (22, 22A) Leuchtdioden mit Dual-in-Line-Gehäuse sind.

10. Sigmoidoskop nach Anspruch 8, wobei die mehreren Leuchtelemente (22, 22A) Leuchtdioden als oberflächenmontiertes Bauelemente sind.

## Revendications

1. Sigmoïdoscope, comprenant :
un corps principal (10) ayant
une tête optique (11) ayant
un canal (111) s'étendant à travers la tête optique (11) ; et
un évidement de positionnement (114) disposé à une partie centrale de la tête optique (11) ;
une poignée (12) reliée à la tête optique (11) et ayant une chambre (121) communiquant avec le canal (111) ; et
un ensemble d'éclairage (20) ayant
une carte de circuits imprimés (21) disposée dans l'évidement de positionnement (114) et ayant
un contour annulaire ; et
un trou central (211) aligné avec le canal (111) ;
de multiples éléments électroluminescents (22, 22A) montés de manière annulaire sur la carte de circuits imprimés (21) à intervalles et reliés électriquement à la carte de circuits imprimés (21) ;
deux fils (24) reliés électriquement à la carte de circuits imprimés (21) ;
deux électrodes (25) reliées respectivement et électriquement aux deux fils (24) ; et
au moins une batterie (30) reliant électriquement les deux électrodes (25),
le sigmoïdoscope étant en outre **caractérisé par le fait qu'**il a un interrupteur tactile (23) monté sur la carte de circuits imprimés (21), relié électriquement à la carte de circuits imprimés (21), et qu'il a un bouton-poussoir (231) s'étendant par-dessus un bord du trou central (211) et étant apte à être pressé par un tube d'insertion (43) d'un ensemble spéculum (40) qui est inséré dans le trou central (211) .

2. Sigmoïdoscope selon la revendication 1, dans lequel le sigmoïdoscope a
un manchon de couplage (13) ayant une flexibilité, aligné avec le canal (111) et ayant une partie de butée annulaire (131) ; et
la partie de butée annulaire (131) est intégrée dans l'évidement de positionnement (114) de la tête optique (11) et est adjacente à la carte de circuits imprimés (21).

3. Sigmoïdoscope selon la revendication 2, dans lequel le sigmoïdoscope a
un oculaire (14) relié de manière pivotante à la tête optique (11) et ayant une partie lentille (142) alignée avec le canal (111) ;
le manchon de couplage (13) est disposé entre l'oculaire (14) et la carte de circuits imprimés (21).

4. Sigmoïdoscope selon la revendication 3, dans lequel
le corps principal (10) est composé, de manière symétrique, de deux coques (1A, 1B).

5. Sigmoïdoscope selon la revendication 4, dans lequel le sigmoïdoscope a
un capuchon (15) recouvrant une extrémité de la poignée (12) distale par rapport à la tête optique (11) et ayant un évidement de réception (151) pour contenir l'au moins une batterie (30) ; et
la poignée (12) a un support d'assemblage (123) pour positionner les deux électrodes (25), le support d'assemblage (123) étant disposé adjacent à la chambre (121) et étant distal par rapport à la tête optique (11).

6. Sigmoïdoscope selon la revendication 5, dans lequel la tête optique (11) a au moins une encoche d'engagement (118) disposée de manière distale par rapport à l'oculaire (14).

7. Sigmoïdoscope selon l'une quelconque des revendications 1 à 6, dans lequel
la tête optique (11) a
une première section ;
une seconde section ayant un diamètre interne plus grand qu'un diamètre interne de la première section ;
une section de liaison (112) disposée entre la première section et la seconde section ; et
une nervure annulaire (113) disposée adjacente à la section de liaison (112) ; et
l'évidement de positionnement (114) est disposé entre la section de liaison (112) et la nervure annulaire (113) .

8. Sigmoïdoscope selon la revendication 7, dans lequel la tête optique (11) a de multiples plaques de positionnement (115) disposées le long de la nervure annulaire à intervalles, et chacune des multiples plaques de positionnement a un côté arrière faisant face à l'oculaire et contre lequel la carte de circuits imprimés est mise en butée.

9. Sigmoïdoscope selon la revendication 8, dans lequel les multiples éléments électroluminescents (22, 22A) sont des diodes électroluminescentes à boîtier à double rangée de connexions.

10. Sigmoïdoscope selon la revendication 8, dans lequel les multiples éléments électroluminescents (22, 22A) sont des diodes électroluminescentes à composant monté en surface.
